# EUROPEAN PATENT SPECIFICATION

(11) **EP 3 164 166 B1**
(45) Date of publication and mention of the grant of the patent: **03.03.2021**
(21) Application number: 15733452.5
(22) Date of filing: 01.07.2015
(51) Int. Cl.: A61L 27/34, A61L 27/52, A61L 29/08, A61L 29/14, A61L 31/10, A61L 31/14

(54) **METHOD FOR PROVIDING A HYDROGEL COATING**
VERFAHREN ZUR BEREITSTELLUNG EINER HYDROGELBESCHICHTUNG
PROCÉDÉ D'OBTENTION D'UN REVÊTEMENT D'HYDROGEL

(30) Priority: 03.07.2014 NL 2013115
(43) Date of publication of application: 10.05.2017
(73) Proprietor: Wellinq Medical B.V., 9351 VC Leek (NL)
(72) Inventor: DIEMEER, Martinus Bernardus Johannes, 1906 AD Limmen (NL)
(74) Representative: Verdijck, Gerardus
(86) International application number: PCT/EP2015/065040
(87) International publication number: WO 2016/001331

(56) References cited:
- US-A- 5 573 934
- BURUK ET AL.: "Polyvinylpyrrolidone-based coatings or polyurethanes-the effect of reagent concentration on their chosen physical properties", CHEMICAL AND PROCESS ENGINNERING, vol. 33, no. 4, 2012, pages 563-571, XP002730167, cited in the application
- BUTRUK ET AL.: "Fabrication of biocompatible hydrogel coatings for implantable medical devices using Fenton-type reaction", MATERIALS SCIENCE AND ENGINNERING C.32, 2012, pages 1601-1609, XP002730168,

## Description

The present invention relates according to a first aspect to a method for providing a hydrogel coating on a polymeric substrate of a medical device. According to a further aspect the present invention relates to a polymeric substrate of a medical device obtainable by the present method. According to yet another aspect the present invention relates to a medical device comprising a polymeric substrate obtainable by the present method.

Polymeric substrates, such as used in medical devices like guiding catheters, balloon catheters, urinary catheters, aspiration catheters, introducer sheaths, guidewires, access systems, thrombectomy devices, stent delivery systems, stent graft delivery systems, and heart valve delivery systems, are commonly provided with a biocompatible hydrophilic coating, or hydrogel, to reduce friction force. This enables a convenient and safe use of such a polymeric substrate when inserted into for example a body cavity, blood vessel, trachea or other catheter.

Typically, such a biocompatible, lubricious hydrophilic coating is adhered to the polymeric surface by ultraviolet (UV) treatment, wherein first a primer coating is applied and cured and subsequently a hydrophilic coating is applied and cured. Compositions of such coatings are known, for example from WO2011/157805.

Difficulties arise in applying the ultraviolet curing method to the catheter lumen. It is desirable for the catheter lumen to have a lubricious surface since this provides a more convenient and safe introduction of for example guide wires through the catheter.

Butruk et al, (Polyvinylpyrrolidone based coatings for polyurethanes - the effect of reagent concentration on their chosen physical properties', Chem. Process Eng., 2012, 33 (4), 563-571*)* discloses a method of coating polyurethane materials with a hydrogel layer to enhance their biocompatibility, wherein the coating is covalently bonded to the polymer substrate. Specifically is disclosed that the polyurethane is first immersed in a toluene priming solution for ten minutes, and subsequently in a water grafting solution for another 15 minutes. Although Butruk et al discloses that biocompatible surfaces can be obtained, there is still a need in the art for a hydrogel coating, or hydrogel coating method, having an improved lubricity and improved resistance to wear because these parameters are important in for example catheter applications. For example, the coating process according to Butruk et al used on polyamide and polyurethane substrates provides coatings which show wear during lubricity testing. Another drawback of the disclosed method is the time consuming immersions of 10 and 15 minutes and the use of a hazardous solvent, i.e. toluene.

In view of the above, it is an object of the present invention, amongst other objects, to provide a method for providing a hydrogel coating on a polymeric substrate of a medical device, having an improved lubricity and resistance to wear.

This objective, amongst other objectives, is met by providing a method according to the appended claim 1.

Specifically, this objective is met by providing a method for providing a hydrogel coating, on a polymeric substrate of a medical device, comprising:
(i) a priming step wherein the polymeric substrate is contacted with a priming solution, comprising a radical source and a crosslinking agent, comprising 1 to 30 wt%, preferably 1 to 20 wt %, more preferably 1 to 10 wt %, most preferably 3 to 8 wt% of a poly (ethylene glycol) diacrylate having an average molecular weight of 300 to 750 Da;
(ii) a grafting step following the priming step wherein the polymeric substrate is contacted with a grafting solution, comprising a polymer or a polymer mixture, wherein the polymer mixture is a mixture of polyvinylpyrrolidone and polyethylene oxide, thereby providing a polymeric substrate of a medical device having a hydrogel coating, or having a hydrophilic coating.

As discussed earlier in the background section of this application, a "hydrogel coating" is a hydrophilic coating, preferably, a biocompatible hydrophilic coating.

The method of the present invention is an easy method for coating polymeric substrates, and especially for difficult to reach substrate parts such as a catheter lumen. Furthermore, the present inventors surprisingly found that by using the present method a smooth, highly lubricious hydrogel coating showing no wear was obtained.

The present polyethylene glycol diacrylate, or poly (ethylene glycol) diacrlyate, or PEGDA, has an average molecular weight of 300 to 750 Da, preferably 550 to 725 Da, more preferably of 575 to 700 Da, most preferably about 575 Da or about 700 Da. The present inventors found that, by adding this cross linker, a highly lubricious and adherent hydrogel coating was obtained.

In a preferred embodiment, the present priming solution does not comprise toluene. Advantageously, the present priming solution does not have to diffuse in the polymeric substrate to swell its surface and to allow the penetration of the priming ingredients. Therefore the present priming solution preferably comprises an alcohol. According to a preferred embodiment, the present priming solution comprises ethanol or isopropanol or butanol, as alcoholic solvent. Using ethanol, isopropanol or butanol as solvent is advantageous since these are nonhazardous ingredients that do not compromise the quality of the hydrogel.

As discussed above, the priming solution of the present invention already comprises the polyethylene glycol diacrylate (PEGDA) cross linker. Preferably, the priming solution additionally comprises a cross linking agent. In preferred embodiments, this further cross linking agent is ethylene glycol dimethacrylate (EGDMA). Preferably, the priming solution comprises 1 to 20 wt % ethylene glycol dimethacrylate (EGDMA) in addition to the polyethylene glycol diacrylate.

In alternative less preferred embodiments, the additional cross linking agent may be bisphenol A ethoxylate diacrylate having EO/phenol = 4 and Mₙ= 688, or trimethylolpropane ethoxylate triacrylate having Mₙ= 912.

The present inventors believe that the polyethylene glycol diacrylate provided in the priming solution may adhere to the surface of the polymeric substrate. In addition, the inventors believe that the polyethylene glycol diacrylate may cross link chains of a polymer provided by the grafting solution, for example, PVP chains.

If an additional cross linking agent such as EGDMA is also provided by the priming solution, this cross linking agent is also thought to adhere to the surface of the polymeric susbtrate and cross link polymer chains.

Preferably, the PEGDA priming solution additionally comprises EDGMA. The presence of the EDGMA is thought to be advantageous as it has a lower molecular weight than PEDGA and so forms smaller, stronger cross links between the polymer chains.

In embodiments of the invention, the priming solution may be provided with a surfactant for better surface wetting. The surfactant can be surfactant BYK-UV 3500 sold by BYK-Chemie and it can be present in the solution at about 0.2 wt%.

The priming solution comprises a radical source, where this radical source can be used to generate reactive radical sites on the polymeric substrate. In a preferred embodiment, the radical source may be cumene hydroperoxide (CHP).

In an alternative embodiment, the method may further comprise a pre-priming step in which a polymeric substrate is contacted with a radical source before it is contacted with the priming solution. Suitable radical sources include peroxide solutions, for example, a hydrogen peroxide solution or a solution of cumene hydroperoxide (CHP). For example, a concentrated 30% hydrogen peroxide solution in water may be applied to the polymeric substrate in the pre-priming step, such as by soaking the polymeric substrate in the hydrogen peroxide solution for 2 to 30 minutes. Preferably, the polymeric susbstrate (for example, a catheter) is cleaned before the hydrogen peroxide is applied. After contacting the polymeric substrate with the hydrogen peroxide solution, the polymeric substrate was wiped dry and processed further with the standard priming and grafting.

In embodiments of the invention in which a solution of cumene hydroperoxide is used as the radical source in the pre-priming step, the pre-priming solution may additionally comprise surfactant such as BYK. For example, the solution may comprise 0.2 wt% BYK-UV 3500. A 40% cumene hydroperoxide solution may be used a pre-priming step,

In an alternative pre-priming step, the polymeric substrate can be exposed to a vapour of 30% hydrogen peroxide solution in a closed container.

The inventors believe that soaking/ exposure to hydrogen peroxide may allow hydrogen peroxide to diffuse into the polymeric substrate, and that this peroxide may diffuse back to the surface during the grafting step where it can react with a reducing agent.

In some embodiments of the invention, the pre-priming step may involve coating (for example, by dipping or sponge coating) the polymeric substrate with an alcohol solution of a radical source. For example, the solution may be an ethanol solution comprising 40 wt% cumene hydroperoxide. In addition, the solution may be provided with a surfactant for better surface wetting. The surfactant can be surfactant BYK-UV 3500 sold by BYK-Chemie and it can be present in the solution at about 0.2% by weight. After coating the polymeric substrate, the coating is allowed to dry. For example, the coating may be air dried for 5 minutes.

After the pre-priming step, priming step (i) of the method will take place. In this priming step (i), the polymeric substrate is contacted with a priming solution comprising polyethylene glycol diacrylate. The surface of the polymeric substrate may be coated (for example, by dipping or sponge coating) with an alcohol (for example, ethanol) solution of the polyethylene glycol diacrylate (PEGDA) and a cross linking agent such as ethylene glycol dimethacrylate (EGDMA). For example, in one embodiment, the solution may be an ethanol solution comprising 20% by weight of each of the polyethylene glycol diacrylate and a cross linking agent such as ethylene glycol dimethacrylate.

In embodiments of the invention, the primer solution may comprise buylacrylate. For example, the primer solution may comprise 2 to 10 wt% buylacrylate. When buylacrylate was added to the solution, it was found to advantageously result in a hydrogel coating that was more flexible and thus less prone to cracking on bending.

In another preferred embodiment, the present priming solution, or primer solution, further comprises alcohol, 1 to 20 wt % or 5 to 15 wt % cumene hydroperoxide and / or 1 to 10 or 1 to 20 wt % ethylene glycol dimethacrylate. Preferably, the present priming solution comprises 10 wt % cumene hydroperoxide, 5 wt % ethylene glycol dimethacrylate and / or 5 wt % polyethylene glycol diacrylate. By using the present priming solution the inventors were able to coat various polymeric substrates, such as polyamide and polyurethane substrates of different hardness, with a hydrogel. Additionally, with the same priming solution the inventors were able to successfully coat chlorinated latex, PVC, polycarbonate, polyimide and PET with a hydrogel.

The grafting solution comprises a polymer. This polymer is grafted to the surface of the polymeric substrate. In embodiments of the invention comprising ethylene glycol dimethacrylate, the polymer of the grafting solution can be cross-linked with the ethylene glycol dimethacrylate. It is thought that the polyethylene glycol diacrylate provided in the priming solution enables this cross-linking process between the polymer chains and the ethylene glycol dimethacrylate.

In a preferred embodiment, the grafting solution may comprise polyvinylpyrrolidone (PVP) or poly(ethylene oxide) (PEO). These polymers are preferably high molecular weight (Mw) polyvinylpyrrolidone and poly(ethylene oxide). For example, the grafting solution may comprise polyvinylpyrrolidone with an average molecular weight of 360 kDa (PVP K-90) (i.e. polyvinylpyrrolidone with a mass average molar mass of M_{w} = 360,000).

Alternatively, the grafting solution may comprise polyethylene oxide with viscosity average molar masses of Mᵥ = 600,000 and Mᵥ = 300,000. Suitable poly(ethylene oxide) can be obtained from Sigma Aldrich. A grafting solution may, for example, comprise 2.5 wt % of the poly(ethylene oxide) with a Mᵥ of 600,000, or 2.5 wt % or 5 wt % of the poly(ethylene oxide) with a Mᵥ of 300,000.

Furthermore, in some embodiments, the grafting solution may comprise a mixture of polyvinylpyrrolidone (PVP) and poly(ethylene oxide) (PEO). For example, the grafting solution may comprise equal volumes of a 5 wt % polyvinylpyrrolidone grafting solution and a grafting solution of 2.5 wt % polyethylene oxide with a Mᵥ of 600,000 or 300,000.

In embodiments of the invention comprising ethylene glycol dimethacrylate in addition to at least one of polyvinylpyrrolidone and polyethylene oxide, at least some of the polyvinylpyrrolidone and/or the polyethylene oxide may be cross-linked by ethylene glycol dimethacrylate.

In preferred embodiments, the grafting solution may additionally comprise a reducing agent. This reducing agent may be iron (II) ions (Fe²⁺). In order to provide Fe²⁺ ions, the grafting solution preferably comprise iron (II) sulphate (FeSO₄). Alternatively, the grafting solution may comprise another iron(II) source such as iron (II) chloride (FeCl₂).

The reducing agent, such as the Fe²⁺ ions, will react with an oxidising agent. This oxidising agent may be a peroxide, for example, cumene hydroperoxide or hydrogen peroxide.

In addition, the grafting solution may comprise a further reducing agent for regenerating the Fe²⁺ ions from the iron (III) ions (Fe³⁺) formed upon the oxidation of the Fe²⁺ ions when they are acting as a reducing agent. This further reducing agent may be ascorbic acid.

Preferably, the grafting solution comprises water, FeSO₄ and ascorbic acid in addition to the polymer (for example, in addition to the polyvinylpyrrolidone and/or polyethylene oxide).

According to a preferred embodiment, the present grafting solution comprises water, 1 to 20 wt %, or 5 to 15 wt % polyvinylpyrrolidone, 0.01 to 10 wt % or 0.01 to 1 wt % FeSO₄ and 0.1 to 10 wt % or 0.1 to 5 wt % ascorbic acid. Preferably, the present grafting solution comprises 5 wt % polyvinylpyrrolidone, 0.4 wt % FeSO₄ and / or 2 wt % ascorbic acid.

According to another preferred embodiment, in the present step (i) the priming solution is contacted with the substrate by sponge coating. A suitable form of sponge coating is to treat the surface of the polymeric substrate with sponge sticks, or sandwich the polymeric substrate with sponge sticks in order to efficiently apply a layer of the priming solution. The friction forces, and thus lubricity, for hydrogel coatings produced by sponge coated priming solutions are comparable to those produced by dip coated priming solutions. Sponge coating of catheters is advantageous over dip coating because it is technologically simpler to implement and it allows leaving proximal parts of the catheter uncoated with a hydrogel thereby enabling more grip during handling of the catheter.

According to another preferred embodiment, present step (i) and / or step (ii) comprises contacting the polymeric substrate with the priming solution and / or with the grafting solution for a time period of less than 4 minutes, preferably for a time period of less than 3 minutes, more preferably for a time period of less than 2 minutes or less than 1 minute. Preferably, the present time of contacting the polymeric substrate with priming solution consists of the time required for coating and drying, i.e. there is no extended time required for indiffusion of the priming solution components into the polymeric substrate. In a further preferred embodiment, the present polymeric substrate is contacted with the present priming solution for a time sufficient to coat the desired parts of the polymeric substrate with priming solution, and / or subsequently the polymeric substrate is dried for a time period of less than 4 minutes, preferably for a time period of less than 3 minutes, more preferably for a time period of less than 2 minutes or less than 1 minute. Thus, according to the present invention a quick and efficient method is provided for applying a low friction hydrogel coating onto a polymeric substrate.

Alternatively, in a preferred embodiment, after the present step (i) of priming, the polymeric substrate contacted with the priming solution is dried in order to remove alcohol from the adhering priming solution. Preferably the present polymeric substrate is dried or air dried, more preferably for a time period of 1 to 3 minutes to allow the removal of alcoholic solvent from the adhering priming solution.

According to yet another preferred embodiment, the present polymeric substrate is selected from the group consisting of silicone polymers, acrylic polymers, natural rubber, polysulfones, polyesters, polyethylenes, polypropylenes, polyamides, polyurethanes, polycarbonates, polyimides, vinyl polymers or in short, any polymer with abstractable hydrogens.

According to yet another preferred embodiment, the present medical device is a catheter such as a guiding catheter, balloon catheter, urinary catheter and / or an aspiration catheter. Alternatively, the present medical device is selected from a catheter, introducer sheaths, guidewires, access systems, thrombectomy devices, stent delivery systems, stent graft delivery systems, and heart valve delivery systems.

More preferably, the present polymeric substrate is the lumen of a catheter, since the catheter's lumen can advantageously be coated with the method of the present invention, to provide a catheter having a hydrogel coating within the lumen.

In yet another preferred embodiment, the present polymeric substrate is rinsed with water after step (ii) of grafting, preferably for a time period of least 1 minute.

According to a further preferred embodiment, the present polymeric substrate is contacted with an alcohol after step (ii), and/or after rinsing with water, in order to remove unbound ingredients from the priming solution. It is advantageous to contact the polymeric substrate after step (ii) with the same alcohol as used in the present priming solution. Contacting with alcohol is advantageous for removing not reacted, or not bounded, ingredients of the priming solution, which enables the polymeric substrate to be used for a medical application, wherein it is important to avoid any contamination during medical use. Preferably, the present polymeric substrate is contacted after step (ii) with ethanol, isopropanol or butanol.

According to another preferred embodiment, the method further comprises a post-grafting step (iii) after step (ii). In this post-grafting step (iii), the hydrogel coating is first rinsed in water, then dipped into a solution of ascorbic acid in water (for example, a 2 wt% solution of ascorbic acid), and then soaked in a peroxide solution, for example, a solution of hydrogen peroxide (such as a 1 wt% hydrogen peroxide solution). In such embodiments of the invention, the ascorbic acid dip is thought to reduce any Fe³⁺ ions present in the hydrogel to Fe²⁺ ions for further reaction with the peroxides in which the polymeric substrate is then soaked. This post-grafting step (iii) of soaking the polymeric substrate in a peroxide solution is thought to promote further cross linking of the hydrogel.

As a final step (, the polymeric substrate will be rinsed in water to remove any residual reactants, for example, hydrogen peroxide.

According to another embodiment of the invention, the method comprises an alternative post-grafting step (iii) to that outlined above. In this alternative post-grafting step (iii) the hydrogel coating is first rinsed with water. Then the coating is rinsed with ethanol in order to remove the water from the coating before allowing the coating to dry, for example, by air drying for around two minutes. Subsequently, the coating is coated with a silica solution.

Preferably, the silica solution comprises an alcohol, PEGDA, a polymer and silica nanopowder. Preferably, this solution comprises ethanol, PEGDA, EGDMA, PVP and silica nanopowder. Most preferably, the ethanol solution comprises 5 wt% PEDGA, 5 wt% EGDMA, 1 wt% PVP K-90 and 1 wt% silica nanopowder.

After the hydrogel coating has been coated with the silica solution, it is allowed to dry. The hydrogel coating is then preferably dipped into a solution of ascorbic acid and iron sulphate. This solution may comprise 0.01 to 10 wt % FeSO₄ and 0.1 to 10 wt % ascorbic acid. Preferably, the solution comprises 0.4 wt % FeSO₄ and 2 wt % ascorbic acid.

The hydrogel coating may then be dipped into a peroxide solution, for example, a 1 wt% solution of hydrogen peroxide.

As above, as a final step, the polymeric substrate will be rinsed in water to remove any residual reactants, for example, hydrogen peroxide.

The silica nanopowder can be obtained as Aerosil R7200 from Evonik. This Aerosil R7200 comprises nanosized fumed silica with a surface modification by methacrylate silane.

The inventors believe that by exposing the hydrogen coating to silica in this post-grafting step (iii), the silica particles are incorporated into the hydrogel coating, for example, through bonding with the acrylate groups of the PEDGA (and/or EGDMA).

The presence of silica in the hydrogel has the advantage of providing improved scratch resistance to the hydrogel coating.

Given the beneficial low friction and improved wear properties of the present polymeric substrate, the present invention relates according to another aspect to a polymeric substrate of a medical device having a hydrogel coating, which polymeric substrate, and / or hydrogel, is obtainable by the method of the present invention. A polymeric substrate obtainable by the present method comprises polyethylene glycol diacrylate on the surface of the polymeric substrate. The polyethylene glycol diacrylate is used in the priming solution to prime the polymeric substrate and traces of the polyethylene glycol diacrylate are present on the surface of the polymeric substrate. Preferably, the present polyethylene glycol diacrylate has an average molecular weight of 300 to 750 Da, preferably 550 to 725 Da, more preferably of 575 to 700 Da, most preferably about 575 Da or about 700 Da.

Further, the present medical device is a catheter. More preferably the present medical device is a catheter wherein polyethylene glycol diacrylate is present on the surface of the lumen of the catheter, or is present in the coating of the catheter. More preferably, the present medical device, or catheter, further comprises ethylene glycol dimethacrylate.

Preferably, the present polymeric substrate of a medical device, or the present medical device, has a friction force of lower than 0.0075 N when a normal force of 1 N is applied. More preferably, the present polymeric substrate of a medical device, or the present medical device, has a friction force of lower than 0.0075 N during the first 30 test cycles on a standard friction tester when a force of 1 N is applied. Preferably wherein a force of 1 N is applied with a pull speed of 250 mm/ min.

The present invention will be further elucidated in an example with reference to figure 1 to 7, wherein:
figure 1 shows the results of a comparative lubricity test of run 1, 4, 8, 12, 16 and 20 of 20 test cycles;
figure 2 shows the results of a lubricity test of a hydrogel according to the invention of run 20, 23, 25, 27 and 30 of test cycles 20 to 30;
figure 3 shows the results of a comparative lubricity test of an UV curable hydrogel coating, showing run 1, 3, 5, 7 and 10 of 10 test cycles;
figure 4 shows the results of a lubricity test of a hydrogel according to an embodiment of the invention in which the grafting solution comprises PEO and PVO;
figure 5 shows the results of a lubricity test of a hydrogel formed by a method comprising a pre-priming step;
figure 6 shows the results of a lubricity test of another embodiment of a hydrogel formed by a method comprising a pre-priming step; and
figure 7 shows the results of a lubricity test of another embodiment of a hydrogel formed by a method comprising a post-grafting step.

### Example (not according to the invention)

### Materials:

Priming solution:
   Cumene hydroperoxide (CHP), ethylene glycol dimethacrylate (EGDMA), poly(ethylene glycol)diacrylate with average molecular weight of 575 Da (PEGDA 575), poly(ethylene glycol)diacrylate with average molecular weight of 700 Da (PEGDA 700), ethanol, isopropanol or butanol as solvent.
Grafting solution:
   Polyvinylpyrrolidone with average molecular weight of 360 kDa (PVP K-90), iron (II) chloride (FeCl₂) or iron (II) sulfate (FeSO₄), ascorbic acid (AA), water as solvent.

### Coating:

Hydrogel coatings on Polyamide (different Vestamid™ blends) and Polyurethane (various Pellethane's™) catheters were fabricated in a two-step coating method i.e. a priming step followed by a grafting step. The catheters were sponge-coated or dip-coated by an ethanol, isopropanol or butanol priming solution containing 10 wt % CHP, 5 wt % EGDMA and 5 wt % PEGDA 575 or 5 wt % PEGDA 700. The samples were air-dried for 1 minute to remove the alcohol from the coating. The samples were then placed in the grafting solution consisting of water containing 5 wt % of PVP K-90, 0.4 wt % FeSO₄ and 2 wt % AA for 1.5 minutes at 25°C to graft a hydrogel to the surface. After the coating procedure, the catheters were flushed with water for 1 minute, followed by soaking in ethanol, isopropanol or butanol. Hydrogel coatings on the lumen of the catheters are applied by the same procedure whereby the coating and cleaning solutions are sucked or pushed in the lumen from a reservoir and the drying of the coating is performed by blowing compressed air or nitrogen through the lumen.

### Testing:

Lubricity tests were performed on an Instron friction tester (Instron Series IX automated materials tester 5544) with the samples immersed in demineralized water. Catheter pieces were attached to the movable load cell of the friction tester using a guide wire inserted into the catheter lumen and clamped between two stationary contact pads. These were made of natural latex rubber. A normal (clamping) force of 1 N was applied to the contact pad/catheter interfaces. The displacement of the load cell was 20 cm. The pull speed was 250 mm/min.

The average friction force (AvFF), as measured during 20 draw-ups, was used to characterize the lubricity and mechanical stability of the coating. The friction forces recorded for the start- and end section were excluded for the determination of the AvFF. An AvFF< 0.05 N is rated as good.

An uncoated Polyamide catheter shows an AvFF of 1.0-1.2 N. An increasing AvFF is an indication of coating wear.

### Comparative hydrogel coating procedure, according to B. Butruk et al.

### (Chem. Process Eng., 2012, 33 (4), 563-571)

### Coating:

Hydrogel coatings on Polyamide (different Vestamid™ blends) and Polyurethane (various Pellethane's™) catheters were fabricated in a two-step dip-coating. The catheters were immersed in a toluene solution containing 10 wt % CHP, 5 wt % EGDMA and dried for 1 minute at 25°C. The samples were then placed in a water solution containing 5 wt % of PVP K-90, 0.4 wt % FeCl₂ and 2 % (w/v) AA for 1.5 minutes at 25°C. After the coating procedure, the catheters were flushed with water for 1 minute, followed by soaking in ethanol, isopropanol or butanol.

### Results

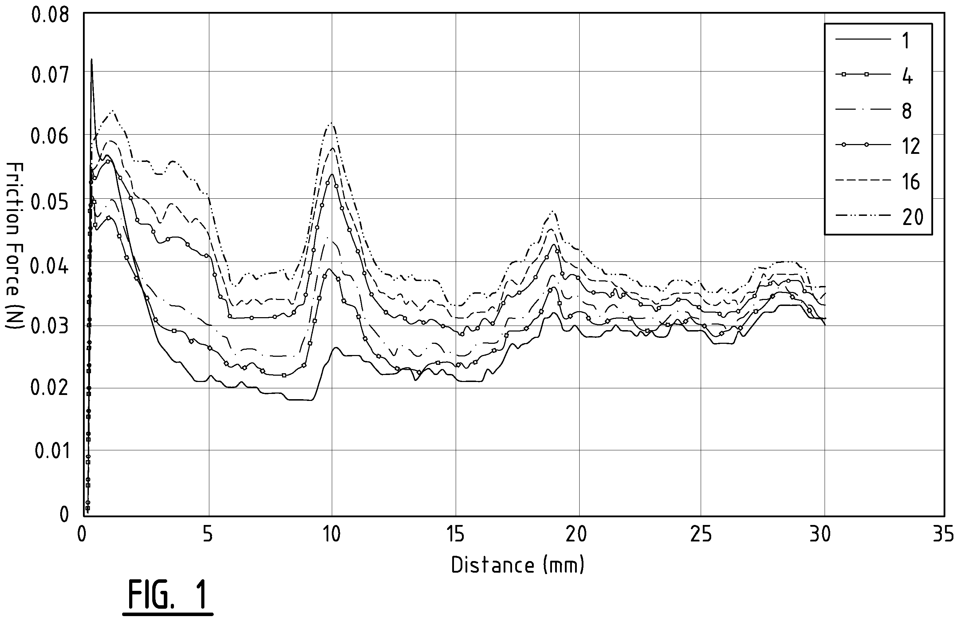

Figure 1 shows the test results of a comparative hydrogel-coated Vestamid (polyamide) catheter according to Butruk et al with shortened priming and grafting times of 1-2 min. The procedure provided a poorly adherent hydrogel coating. The same results were found when toluene in the priming solution is replaced by less hazardous ethanol, isopropanol or butanol. Figure 1 shows that during the 20 test cycles, the friction force (AvFF) increases from 0.025 to 0.035 N, which indicates coating wear as a consequence of the poor adhesion.

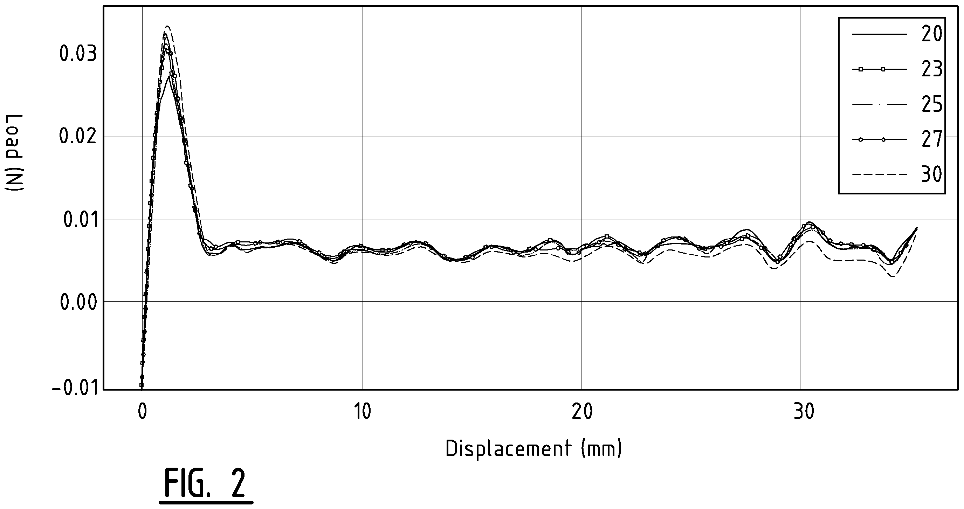

Figure 2 shows the test results for a Vestamid (polyamide) catheter coated according to the procedure of the present invention after 20-30 testcycles. The AvFF remains low at 0.0075 N, and thus the present method provides an improve lubricity over the method of Butruk et al. Further, Figure 2 shows no increase in friction force, which indicates the advantageous wear properties of the coating. Surprisingly, by adding PEGDA 575 or PEGDA 700 to the priming solution, a smooth, highly lubricious hydrogel coating showing no wear was obtained even when priming and grafting times as short as 1-2 minutes were used (see fig. 2). Moreover, this also allowed replacing the toluene of Butruk's priming solution by ethanol, isopropanol or butanol without compromising the quality of the coating. The same results were found for polyurethane catheters.

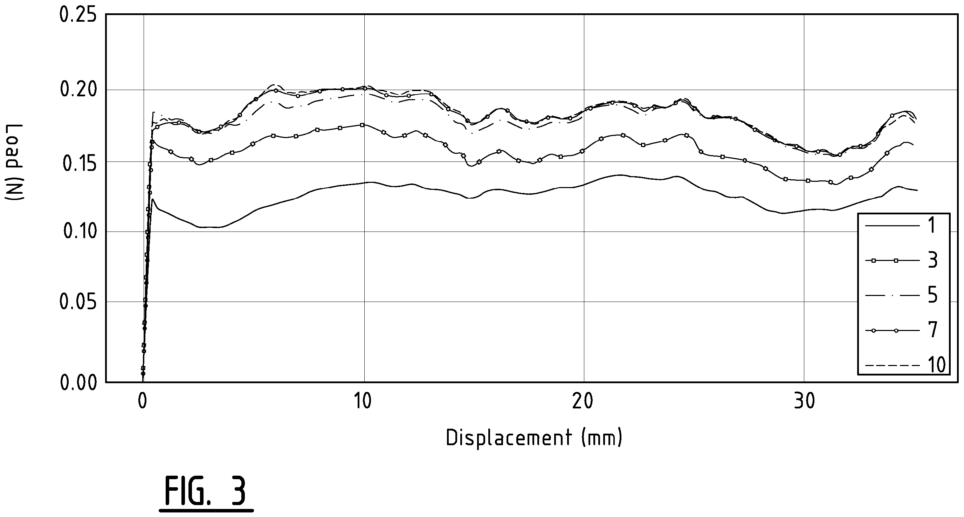

To appreciate the quality of the hydrogel provided with the present method it can be compared with a best-in-class commercially available UV-curable hydrogel coating (DSM ComfortCoat® according to WO2011/157805). Figure 3 shows the evolution of the friction force during 10 draw-ups of the Vestamid catheter provided with this coating. It shows signs of wear during the first 5 draw-ups with an increase of AvFF from about 0.125 N to a final level of about 0.157 N, which is more than an order of magnitude higher than the value as measured for the hydrogel coating provided according to the present invention.

### Example 2

### Materials:

Priming solution:
   The solution comprises 20% PEGDA and 20% EGDMA in ethanol.
Grafting solution:
   The solution comprises a 1/1 (volume) mixture of:
   a first solution containing 5 wt % of PVP K-90, 0.4 wt % FeSO₄ and 2 wt % AA in water, and
   a second solution containing 2.5 wt % of polyethylene oxide with viscosity average molar mass of Mᵥ = 300,000, 0.4 wt % FeSO₄ and 2 wt % AA in water

### Coating:

The hydrogel coatings were fabricated on Vestamid™ (polyamide) catheters in a three-step coating method. In addition to the priming and grafting steps of Example 1, this three-step method comprises a pre-priming step.

In the pre-priming step, the Vestamid™ catheters were sponge-coated or dip-coated by a solution of ethanol containing 40% CHP and 0.2% BYK-UV 3500 and dried in air for 5 minutes.

Next, the catheters were sponge-coated or dip-coated by a priming solution with 10% EGDMA and 10% PEGDA. The samples were then air-dried for 1 minute to remove the alcohol from the coating.

The samples were then placed in the grafting solution for 1.5 minutes at 25°C to graft a hydrogel to the surface.

After the coating procedure, the catheters were flushed with water for 1 minute, followed by soaking in ethanol, isopropanol or butanol.

### Testing:

Lubricity tests were performed in the same way as for Example 1.

### Results

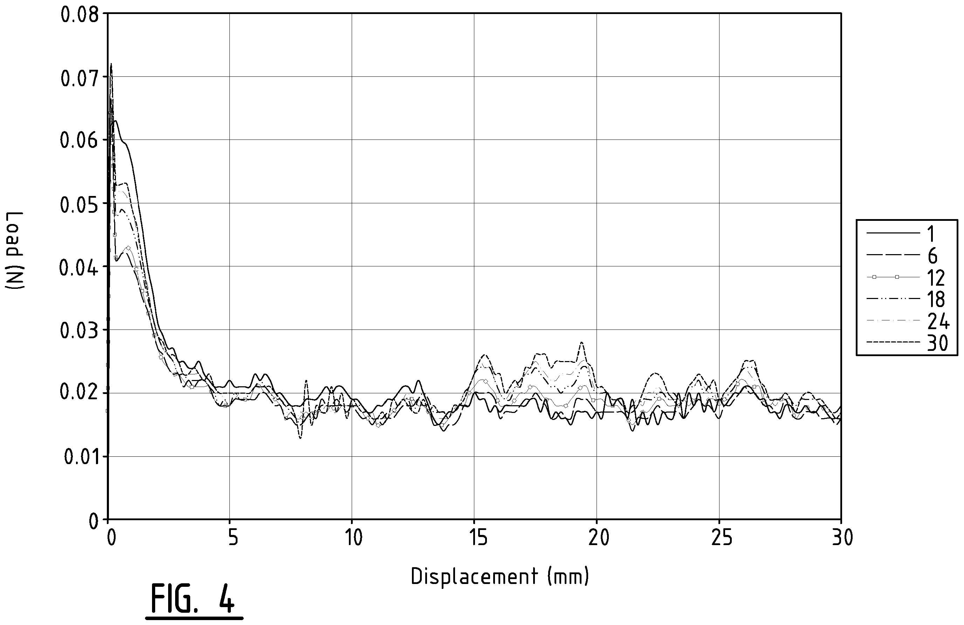

Figure 4 shows the test results of a hydrogel-coated Vestamid (polyamide) catheter coated according to the procedure of Example 2 after 30 test cycles. The friction force (AvFF) remains low at 0.02 N. Further, Figure 4 shows no increase in friction force, which indicates the advantageous wear properties of the coating.

### Example 3

### Materials:

Priming solution:
   An ethanol, isopropanol or butanol priming solution containing 10 wt % EGDMA and 10 wt % PEGDA 575 or 5 wt % PEGDA 700.
Grafting solution:
   Same as that used in Example 2.

### Coating:

The hydrogel coatings were fabricated on Vestamid™ (polyamide) catheters in a three-step coating method. Like the method of Example 2, the method of Example 3 comprises a pre-priming step.

In the pre-priming step, the Vestamid™ catheters were firstly enclosed in a container with 30% hydrogen peroxide vapour for 20 minutes.

The catheters were subsequently sponge-coated or dip-coated by the priming solution. The samples were then air-dried for 1 minute to remove the alcohol from the coating.

The samples were then placed in the grafting solution for 1.5 minutes at 25°C to graft a hydrogel to the surface.

After the coating procedure, the catheters were flushed with water for 1 minute, followed by soaking in ethanol, isopropanol or butanol.

### Testing:

Lubricity tests were performed in the same way as for Example 1.

### Results

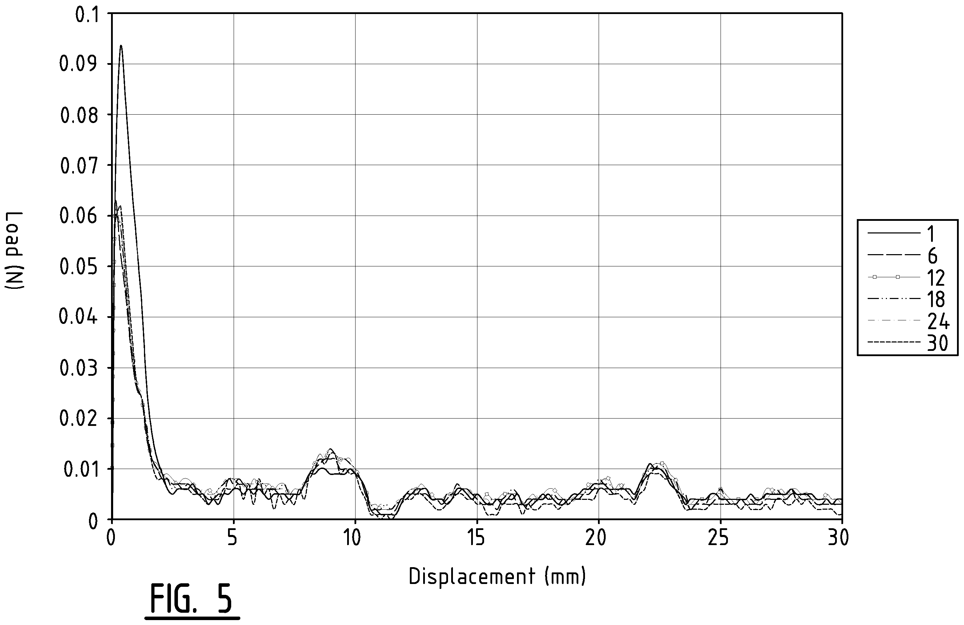

Figure 5 shows the test results of a hydrogel-coated Vestamid catheter coated according to the procedure of Example 3 after 30 test cycles. The AvFF remains low at below 0.01 N. Further, Figure 5 shows no increase in friction force, which indicates the advantageous wear properties of the coating.

### Example 4

### Materials:

Priming solution:
   Same as that used in Example 3.
Grafting solution:
   Same as that used in Example 2.

### Coating:

As with Example 3, the hydrogel coatings were fabricated on Vestamid™ (polyamide) catheters in a three-step coating method.

In the pre-priming step, the Vestamid™ catheters were dipped into a 30 wt% hydrogen peroxide solution for 5 minutes.

The catheters were subsequently sponge-coated or dip-coated by the priming solution. The samples were then air-dried for 1 minute to remove the alcohol from the coating.

The samples were then placed in the grafting solution for 1.5 minutes at 25°C to graft a hydrogel to the surface.

After the coating procedure, the catheters were flushed with water for 1 minute, followed by soaking in ethanol, isopropanol or butanol.

### Testing:

Lubricity tests were performed in the same way as for Example 1.

### Results

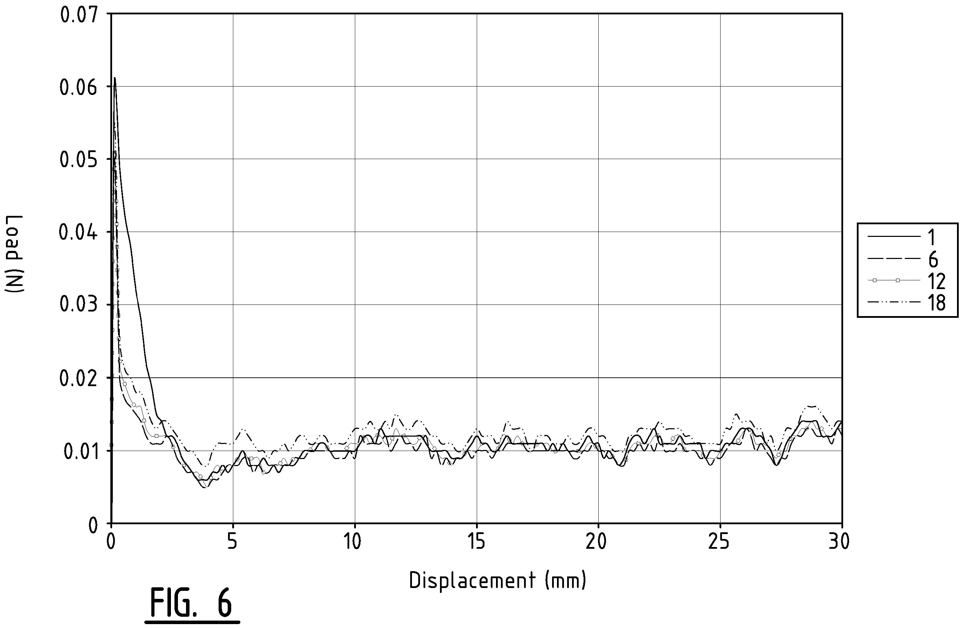

Figure 6 shows the test results of a hydrogel-coated Vestamid catheter coated according to the procedure of Example 4 after 30 test cycles. The AvFF remains low at below 0.02 N. Further, Figure 6 shows no increase in friction force, which indicates the advantageous wear properties of the coating.

### Example 5 (not according to the invention)

### Materials:

Priming solution:
   Same as that used in Example 2.
Grafting solution:
   Same as that used in Example 1.

### Coating:

Hydrogel coatings were fabricated on Vestamid™ (polyamide) catheters in a four-step coating method. In addition to the standard priming and grafting steps, the method comprises a pre-priming step like Example 2. The method of Example 5 also comprises a post-grafting step.

After the grafting step, the catheters were washed with water followed by ethanol. After drying in air for 2 minutes, the hydrogel coating was overcoated with a solution of ethanol containing 5 wt% EGDMA, 5 wt% PEGDA, 1 wt% PVP K-90 and 1 wt% silica nanopowder (Aerosil R7200 produced by Evonik). The coating was then left to dry before it was dipped in a solution containing 2 wt % ascorbic acid and 0.4 wt % FeSO₄. Subsequently, the catheters were soaked in a solution of 1 wt% hydrogen peroxide to cross-link the coating. This crosslinking is initiated by the redox reaction of the peroxide with the Fe(II) ions.

After the coating procedure, the catheters were flushed with water for 1 minute, followed by soaking in ethanol, isopropanol or butanol.

### Testing:

Lubricity tests were performed in the same way as for Example 1.

### Results

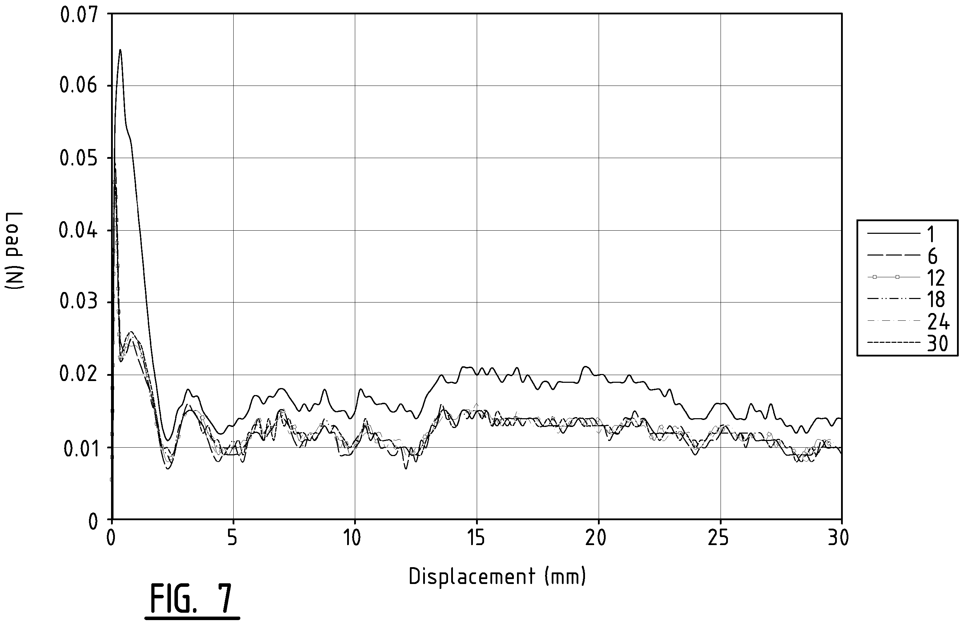

Figure 7 shows the test results of a hydrogel-coated Vestamid (polyamide) catheter coated according to the procedure of Example 5 after 21 testcycles. The AvFF remains low at 0.02 N. Further, Figure 7 shows no increase in friction force, which indicates the advantageous wear properties of the coating.

## Claims

1. Method for providing a hydrogel coating on a polymeric substrate of a medical device comprising:
(i) a priming step wherein the polymeric substrate is contacted with a priming solution, comprising a radical source and a crosslinking agent, comprising 1 to 30 wt % of a polyethylene glycol diacrylate having an average molecular weight of 300 to 750 Da;
(ii) a grafting step following the priming step wherein the polymeric substrate is contacted with a grafting solution, comprising a polymer mixture, wherein the polymer mixture is a mixture of polyvinylpyrrolidone and polyethylene oxide, thereby providing a polymeric substrate of a medical device having a hydrogel coating.

2. Method according to claim 1, wherein the polyethylene glycol diacrylate has an average molecular weight of 550 to 725 Da, preferably of 575 Da or 700 Da.

3. Method according to claim 1 or claim 2, wherein the priming solution comprises a radical source, wherein the radical source is preferably cumene hydroperoxide.

4. Method according to any of the claims 1 to 3, wherein the priming solution comprises alcohol.

5. Method according to any of the claims 1 to 4, wherein the priming solution comprises ethylene glycol dimethacrylate.

6. Method according to any of the claims 1 to 5, wherein the priming solution comprises alcohol, 1 to 20 wt % cumene hydroperoxide and 1 to 20 wt % ethylene glycol dimethacrylate.

7. Method according to any of the claims 1 to 6, wherein the priming solution comprises buylacrylate, preferably 2 to 10 wt % buylacrylate.

8. Method according to any of the claims 1 to 7, wherein the grafting solution comprises one or more of: polyvinylpyrrolidone preferably polyvinylpyrrolidone with an average molecular mass of 360 kDa; polyethylene oxide; a reducing agent, wherein preferably the reducing agent is Fe²⁺ ions and preferably additional reducing agent, wherein preferably the additional reducing agent is ascorbic acid; and/or water, 1 to 20 wt % polyvinylpyrrolidone, 0.01 to 10 wt % FeSO₄ and 0.1 to 10 wt % ascorbic acid.

9. Method according to any of the claims 1 to 8, wherein in step (i) the priming solution is contacted with the substrate by sponge coating.

10. Method according to any of the claims 1 to 9, wherein step (i) and / or step (ii) comprises contacting the polymeric substrate with the priming solution and / or the grafting solution for a time period of less than 4 minutes, preferably for a time period of less than 3 minutes, more preferably for a time period of less than 1 or 2 minutes.

11. Method according to any of the claims 1 to 10, wherein the priming solution comprises ethanol, isopropanol or butanol as alcohol.

12. Method according to any of the claims 1 to 11, wherein after step (i) the polymeric substrate contacted with the priming solution is dried in order to remove alcohol from the adhering priming solution.

13. Method according to any of the claims 1 to 12, wherein the polymeric substrate is selected from the group consisting of. silicone polymers, acrylic polymers, natural rubber, polysulfones, polyesters, polyethylenes, polypropylenes, polyamides, polyurethanes, polycarbonates, polyimides, vinyl polymers and polymer with abstractable hydrogens.

14. Method according to any of the claims 1 to 13, wherein the medical device is a catheter and/or wherein the polymeric substrate is the lumen of the catheter.

15. Method according to any of the claims 1 to 14, wherein after step (ii) the polymeric substrate is rinsed with water, preferably for at least 1 minute.

16. Method according to any of claims 1 to 15, wherein the method further comprises a post-grafting step (iii) after step (ii), wherein the post-grafting step (iii) comprises first rinsing the hydrogel coating with water, then dipping the hydrogel coating into a solution of ascorbic acid in water, and then soaking the hydrogel coating in a peroxide solution, for example, a hydrogen peroxide solution, or wherein the method further comprises a post-grafting step (iii) after step (ii), wherein the post-grafting step (iii) comprises first rinsing the hydrogel coating water, then rinsing the hydrogel coating with ethanol before allowing the coating to dry and subsequently coating the hydrogel coating with a silica solution,
wherein preferably the silica solution comprises 5 wt% PEDGA, 5 wt% EGDMA, 1 wt% PVP K-90 and 1 wt% silica nanopowder.

17. Method according to any of the claims 1 to 16, wherein after step (ii) the polymeric substrate is contacted with an alcohol in order to remove unbound ingredients from the priming solution.

18. Method according to any of the claims 1 to 17, wherein the method further comprises a pre-priming step before step (i), wherein the pre-priming step comprises contacting the polymeric substrate with a radical source before the polymeric substrate is contacted with the priming solution, wherein preferably the radical source is hydrogen peroxide solution or hydrogen peroxide vapour or cumene hydroperoxide solution.

19. Polymeric substrate of a medical device having a hydrogel coating obtainable by the method according to any of the claims 1 to 18.

20. Medical device comprising on a polymeric substrate polyethylene glycol diacrylate, preferably polyethylene glycol diacrylate having an average molecular weight of 500 to 750 Da, preferably further comprising polyvinylpyrrolidone and / or ethylene glycol dimethacrylate, obtainable by the method according to any of the claims 1 to 19.

## Patentansprüche

1. Verfahren zum Vorsehen einer Hydrogelbeschichtung auf einem Polymersubstrat einer medizinischen Vorrichtung, das aufweist:
(i) einen Grundierungsschritt, in dem das Polymersubstrat mit einer Grundierungslösung in Kontakt gebracht wird, die eine Radikalquelle und ein Vernetzungsmittel aufweist, das 1 bis 30 Gewichtsprozent eines Polyethylen-Glykol-Diacrylats mit einem durchschnittlichen Molekulargewicht von 300 bis 750 Da aufweist;
(ii) einen Aufpropfungsschritt, der auf den Grundierungsschritt folgt, wobei das Polymersubstrat mit einer Aufpfropfungslösung in Kontakt gebracht wird, die eine Polymermischung aufweist, wobei die Polymermischung eine Mischung aus Polyvinylpyrrolidon und Polyethylenoxid ist, wodurch ein Polymersubstrat einer medizinischen Vorrichtung vorgesehen wird, die eine Hydrogelbeschichtung aufweist.

2. Verfahren nach Anspruch 1, wobei das Polyethylen-Glykol-Diacrylat ein durchschnittliches Molekulargewicht von 550 bis 725 Da, vorzugsweise 550 Da oder 700 Da hat.

3. Verfahren nach Anspruch 1 oder 2, wobei die Grundierungslösung eine Radikalquelle aufweist, wobei die Radikalquelle vorzugsweise Cumolhydroperoxid ist.

4. Verfahren nach einem der Ansprüche 1 bis 3, wobei die Grundierungsschicht Alkohol aufweist.

5. Verfahren nach einem der Ansprüche 1 bis 4, wobei die Grundierungsschicht EthylenGlykol-Dimetharcylat aufweist.

6. Verfahren nach einem der Ansprüche 1 bis 5, wobei die Grundierungslösung Alkohol, 1 bis 20 Gewichtsprozent Cumolhydroperoxid und 1 bis 20 Gewichtsprozent Ethylen-GlykolDimethacrylat aufweist.

7. Verfahren nach einem der Ansprüche 1 bis 6, wobei die Grundierungslösung Butylacrylat, vorzugsweise 2 bis 10 Gewichtsprozent Butylacrylat aufweist.

8. Verfahren nach einem der Ansprüche 1 bis 7, wobei die Aufpfropfungslösung eines oder mehrere aufweist von:
Polyvinylpyrrolidon, vorzugsweise Polyvinylpyrrolidon mit einer durchschnittlichen Molekularmasse von 360 kDa; Polyethylenoxid; ein Reduzierungsmittel, wobei das Reduzierungsmittel vorzugsweise FE2+-Ionen sind und vorzugsweise ein weiteres Reduzierungsmittels ist, wobei das weitere Reduzierungsmittel vorzugsweise Ascorbinsäure und/oder Wasser ist; 1 bis 20 Gewichtsprozent Polyvinylpyrrolidon, 0,01 bis 10 Gewichtsprozent und 0,1 bis 10 Gewichtsprozent Ascorbinsäure.

9. Verfahren nach einem der Ansprüche 1 bis 8, wobei in Schritt (i) die Grundierungslösung mit dem Substrat durch Schwammbeschichtung im Kontakt gebracht wird.

10. Verfahren nach einem der Ansprüche 1 bis 9, wobei der Schritt (i) und/oder der Schritt (ii) aufweisen, dass das Polymersubstrat mit der Grundierungslösung und/oder der Aufpfropfungslösung für eine Zeitspanne von weniger als 4 Minuten in Kontakt gebracht wird, vorzugsweise für eine Zeitspanne von weniger als 3 Minuten, noch bevorzugter für eine Zeitspanne von weniger als 1 oder 2 Minuten.

11. Verfahren nach einem der Ansprüche 1 bis 10, wobei die Grundierungsschicht Ethanol, Isopropanol oder Butanol als Alkohol aufweist.

12. Verfahren nach einem der Ansprüche 1 bis 11, wobei nach Schritt (i) das Polymersubstrat, das mit der Grundierungslösung in Kontakt gebracht wurde, getrocknet wird, um den Alkohol aus der anhaftenden Grundierungslösung zu entfernen.

13. Verfahren nach einem der Ansprüche 1 bis 12, wobei das Polymersubstrat ausgewählt ist aus der Gruppe bestehend aus Siliziumpolymeren, Acrylpolymeren, natürlichem Kautschuk, Polysulfonen, Polyestern, Polyethylenen, Polypropylenen, Polyamiden, Polyurethanen, Polycarbonaten, Polyimiden, Vinylpolymeren und Polymeren mit abspaltbaren Wasserstoffatomen.

14. Verfahren nach einem der Ansprüche 1 bis 13, wobei die medizinische Vorrichtung ein Katheter ist und/oder wobei das Polymersubstrat das Lumen des Katheters ist.

15. Verfahren nach einem der Ansprüche 1 bis 14, wobei nach Schritt (ii) das Polymersubstrat mit Wasser gespült wird, vorzugsweise für wenigstens 1 Minute.

16. Verfahren nach einem der Ansprüche 1 bis 15, wobei das Verfahren weiterhin einen Schritt nach dem Aufpfropfen (iii) nach Schritt (ii) aufweist, wobei der Schritt nach dem Aufpfropfen (iii) ein erstes Spülen der Hydrogelbeschichtung mit Wasser, dann das Eintauchen der Hydrogelbeschichtung in eine Lösung aus Ascorbinsäure in Wasser, und dann das Einweichen der Hydrogelbeschichtung in eine Peroxidlösung, zum Beispiel eine Hydrogenperoxidlösung, aufweist, oder wobei das Verfahren weiterhin einen Schritt nach dem Aufpfropfen (iii) nach Schritt (ii) aufweist, wobei der Schritt nach dem Aufpfropfen (iii) aufweist ein erstes Spülen des Hydrogelbeschichtungswassers, dann das Spülen der Hydrogelbeschichtung mit Ethanol, bevor die Hydrogelbeschichtung trocknen gelassen wird und danach Beschichten der Hydrogelbeschichtung mit einer Siliziumdioxidlösung, wobei die Siliziumdioxidlösung vorzugsweise 5 Gewichtsprozent PEDGA, 5 Gewichtsprozent EGDMA, 1 Gewichtsprozent PVP K-90 und 1 Gewichtsprozent Siliziumdioxid-Nanopulver aufweist.

17. Verfahren nach einem der Ansprüche 1 bis 16, wobei nach Schritt (ii) das Polymersubstrat mit Alkohol in Kontakt gebracht wird, um die ungebundenen Inhaltsstoffe aus der Grundierungslösung zu entfernen.

18. Verfahren nach einem der Ansprüche 1 bis 17, wobei das Verfahren weiterhin einen Vorgrundierungsschritt vor Schritt (i) aufweist, wobei der Vorgrundierungsschritt das in Kontaktbringen des Polymersubstrats mit einer Radikalquelle aufweist, bevor das Polymersubstrat mit der Grundierungslösung in Kontakt gebracht wird, wobei die Radikalquelle vorzugsweise eine Hydrogenperoxidlösung oder ein Hydrogenperoxiddampf oder eine Cumolhydroperoxidlösung ist.

19. Polymersubstrat einer medizinischen Vorrichtung, die eine Hydrogelbeschichtung aufweist, die durch das Verfahren nach einem der Ansprüche 1 bis 18 erhaltbar ist.

20. Medizinische Vorrichtung, die ein Polymersubstrat-Polyethylen-Glykol-Diacrylat aufweist, vorzugsweise ein Polyethylen-Glykol-Diacrylat, das ein durchschnittliches Molekulargewicht von 500 bis 750 Da hat, vorzugsweise weiterhin Polyvinylpyrrolidon und/oder Ethylen-GlykolDimethacrylat aufweisend, das aus dem Verfahren nach einem der Ansprüche 1 bis 19 erhaltbar ist.

## Revendications

1. Procédé de formation d'un revêtement d'hydrogel sur un substrat polymère d'un dispositif médical comprenant :
(i) une étape d'amorçage dans laquelle le substrat polymère est mis en contact avec une solution d'amorçage, comprenant une source de radicaux et un agent de réticulation, comprenant de 1 à 30 % en poids d'un di-acrylate de polyéthylène glycol présentant un poids moléculaire moyen de 300 à 750 Da ;
(ii) une étape de greffe à la suite de l'étape d'amorçage dans laquelle le substrat polymère est mis en contact avec une solution de greffe, comprenant un mélange de polymères, dans lequel le mélange de polymères est un mélange de polyvinylpyrrolidone et d'oxyde de polyéthylène, formant ainsi un substrat polymère d'un dispositif médical comportant un revêtement d'hydrogel.

2. Procédé selon la revendication 1, dans lequel le diacrylate de polyéthylène glycol présente un poids moléculaire moyen de 550 à 725 Da, de préférence, égal à 575 Da ou à 700 Da.

3. Procédé selon la revendication 1 ou 2, dans lequel la solution d'amorçage comprend une source de radicaux, dans lequel la source de radicaux est, de préférence, de l'hydroperoxyde de cumène.

4. Procédé selon l'une quelconque des revendications 1 à 3, dans lequel la solution d'amorçage comprend de l'alcool.

5. Procédé selon l'une quelconque des revendications 1 à 4, dans lequel la solution d'amorçage comprend du diméthacrylate d'éthylène-glycol.

6. Procédé selon l'une quelconque des revendications 1 à 5, dans lequel la solution d'amorçage comprend de l'alcool, de 1 à 20 % en poids d'hydroperoxyde de cumène et de 1 à 20 % en poids de diméthacrylate d'éthylène-glycol.

7. Procédé selon l'une quelconque des revendications 1 à 6, dans lequel la solution d'amorçage comprend de l'acrylate de butyle, de préférence, de 2 à 10% d'acrylate de butyle.

8. Procédé selon l'une quelconque des revendications 1 à 7, dans lequel la solution de greffe comporte un ou plusieurs parmi :
le polyvinylpyrrolidone, de préférence, du polyvinylpyrrolidone présentant une masse moléculaire moyenne de 360 kDa ;
l'oxyde de polyéthylène ; un agent réducteur, dans lequel, de préférence, l'agent réducteur est formé d'ions Fe2+ et, de préférence, un agent réducteur supplémentaire, dans lequel, de préférence, l'agent réducteur supplémentaire est de l'acide ascorbique et/ou de l'eau ;
de 1 à 20% en poids de polyvinylpyrrolidone, de 0,01 à 10% en poids de et de 0,1 à 10% en poids d'acide ascorbique.

9. Procédé selon l'une quelconque des revendications 1 à 8, dans lequel, à l'étape (i), la solution d'amorçage est mise en contact avec le substrat par revêtement à l'éponge.

10. Procédé selon l'une quelconque des revendications 1 à 9, dans lequel l'étape (i) et/ou l'étape (ii) comprennent la mise en contact du substrat polymère avec la solution d'amorçage et/ou la solution de greffe pendant une période inférieure à 4 minutes, de préférence, pendant une période inférieure à 3 minutes, plus préférablement, pendant une période inférieure à 1 ou 2 minutes.

11. Procédé selon l'une quelconque des revendications 1 à 10, dans lequel la solution d'amorçage comprend de l'éthanol, de l'isopropanol ou du butanol en tant qu'alcool.

12. Procédé selon l'une quelconque des revendications 1 à 11, dans lequel, après l'étape (i), le substrat polymère mis en contact avec la solution d'amorçage est séché afin d'éliminer l'alcool de la solution d'amorçage adhérente.

13. Procédé selon l'une quelconque des revendications 1 à 12, dans lequel le substrat polymère est sélectionné à partir du groupe constitué par des polymères de silicone, des polymères acryliques, du caoutchouc naturel, des polysulfones, des polyesters, des polyéthylènes, des polypropylènes, des polyamides, des polyuréthanes, des polycarbonates, des polyimides, des polymères vinyliques et un polymère comportant des hydrogènes extractibles.

14. Procédé selon l'une quelconque des revendications 1 à 13, dans lequel le dispositif médical est un cathéter et/ou dans lequel le substrat polymère est la lumière du cathéter.

15. Procédé selon l'une quelconque des revendications 1 à 14, dans lequel, après l'étape (ii) le substrat polymère est rincé à l'eau, de préférence, pendant au moins 1 minute.

16. Procédé selon l'une quelconque des revendications 1 à 15, dans lequel le procédé comprend, en outre, une étape post-greffe (iii) après l'étape (ii), dans lequel l'étape post-greffe (iii) comprend un premier rinçage du revêtement d'hydrogel à l'eau, puis le trempage du revêtement d'hydrogel dans une solution aqueuse d'acide ascorbique, et ensuite, l'imprégnation du revêtement d'hydrogel dans une solution de peroxyde, par exemple, une solution de peroxyde d'hydrogène, ou dans lequel le procédé comprend, en outre, une étape post-greffe (iii) après l'étape (ii), dans lequel l'étape post-greffe (iii) comprend un premier rinçage du revêtement d'hydrogel à l'eau, puis un rinçage du revêtement d'hydrogel avec de l'éthanol avant de faire sécher le revêtement et consécutivement, l'enrobage du revêtement d'hydrogel avec une solution de silice,
dans lequel, de préférence, la solution de silice comprend 5 % en poids de PEDGA, 5 % en poids d'EGDMA, 1 % en poids de PVP K-90 et 1 % en poids de nanopoudre de silice.

17. Procédé selon l'une quelconque des revendications 1 à 16, dans lequel, après l'étape (ii), le substrat polymère est mis en contact avec de l'alcool dans le but d'éliminer des ingrédients non liés de la solution d'amorçage.

18. Procédé selon l'une quelconque des revendications 1 à 17, dans lequel le procédé comprend, en outre, une étape de pré-amorçage avant l'étape (i), dans lequel l'étape de pré-amorçage comprend la mise en contact du substrat polymère avec une source de radicaux avant que le substrat polymère ne soit mis en contact avec la solution d'amorçage, dans lequel, de préférence, la source de radicaux est une solution de peroxyde d'hydrogène ou de vapeur de peroxyde d'hydrogène ou une solution d'hydroperoxyde de cumène.

19. Substrat polymère d'un dispositif médical présentant un revêtement d'hydrogel pouvant être obtenu par le procédé selon l'une quelconque des revendications 1 à 18.

20. Dispositif médical comprenant, sur un substrat polymère, du diacrylate de polyéthylène glycol, de préférence, du diacrylate de polyéthylène glycol présentant un poids moléculaire moyen de 500 à 750 Da, comprenant, en outre, de préférence, du polyvinylpyrrolidone et/ou du diméthacrylate d'éthylène-glycol, pouvant être obtenu par le procédé selon l'une quelconque des revendications 1 à 19.
